# EUROPÄISCHE PATENTSCHRIFT

(11) **EP 1 210 047 B1**
(45) Veröffentlichungstag und Bekanntmachung des Hinweises auf die Patenterteilung: **09.02.2005**
(21) Anmeldenummer: 00929382.0
(22) Anmeldetag: 19.04.2000
(51) Int. Cl.: A61F 13/38, A61F 11/00

(54) **OHRWATTESTÄBCHEN UND HOSPITALSTÄBCHEN**
COTTON BUDS AND SWABS FOR MEDICAL USE
COTONS-TIGES ET BATONNETS POUR USAGE EN MILIEU HOSPITALIER

(30) Priorität: 22.04.1999 DE 19918242
(43) Veröffentlichungstag der Anmeldung: 05.06.2002
(73) Patentinhaber: Q-Plast GmbH & Co., 46446 Emmerich (DE)
(72) Erfinder: Prager, Robert, 46446 Emmerich (DE)
(74) Vertreter: Durm, Frank, Dipl.-Ing.
(86) Internationale Anmeldenummer: PCT/EP2000/003529
(87) Internationale Veröffentlichungsnummer: WO 2000/064397

(56) Entgegenhaltungen:
- DE-B- 1 183 641
- DE-B- 1 188 775
- FR-A- 2 129 143

## Beschreibung

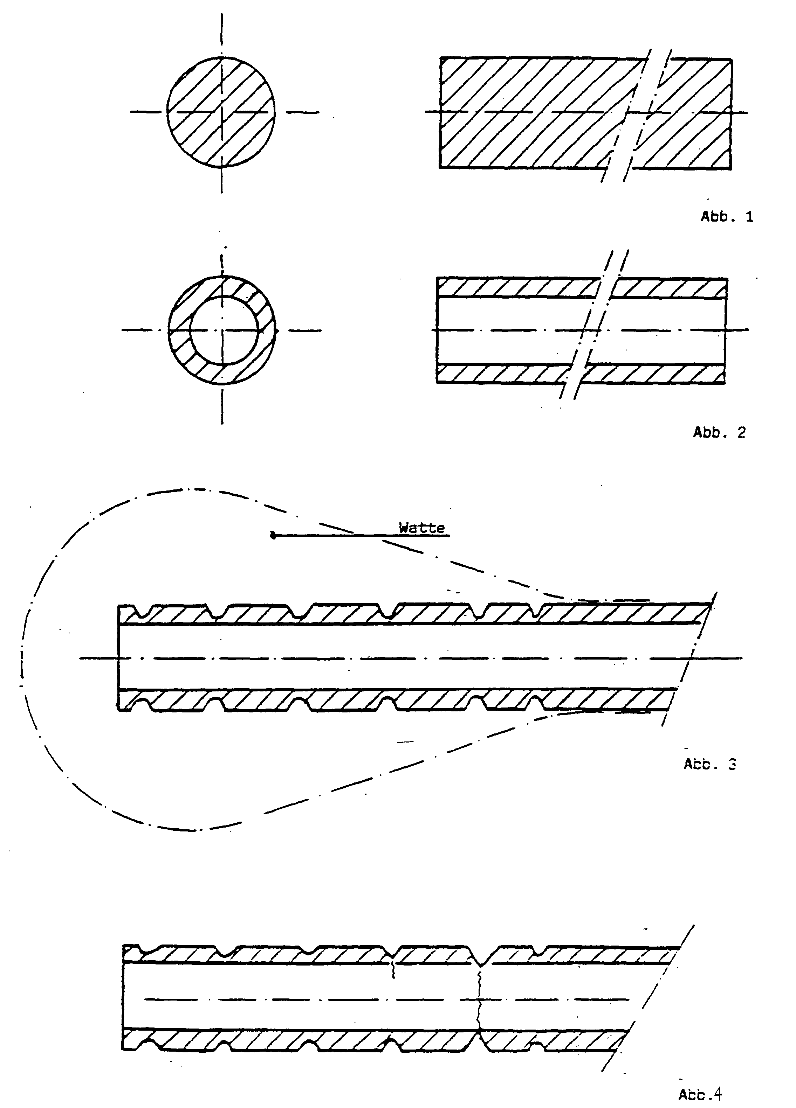

Kunststoffstiele für Ohrwattestäbchen und Hospitalstäbchen werden hauptsächlich als Strang extrudiert und anschließend auf Länge geschnitten, selten als Spritzgußteile hergestellt. Die Spritzgußstäbchen sind massiv **(Abb. 1)** und haben deshalb ein hohes Gewicht.

Für eine ähnliche Anwendung, einen Interdentalreiniger, beschreibt das DE-Patent DE 196 42 431 A 1 einen länglichen, stabförmigen Träger aus einem ersten Material, der in Teilbereichen seiner Oberfläche von zumindest einer Ein- oder Auflage aus einem zweiten Material überdeckt ist, das weicher ist als das erste Material. Die dort beschriebene Querschnittsform des Trägers ist im wesentlichen beliebig, insbesondere oval, rund, dreieckig oder abgeflacht. Auch die Querschnittsform der Ein- oder Auflage wird dort im wesentlichen beliebig gewählt, wobei auch insoweit insbesondere ovale, runde, abgeflachte oder dreieckige Formen Verwendung finden. Diese Methode erfordert jedoch den Einsatz mehrerer Materialien. Auch ist das Gewicht durch den hohen Materialeinsatz relativ hoch.

Das DE-Patent DE 37 09 497 A 1 behandelt ein Tupferstäbchen für einen Mundtupfer, der durch einen Stützstab stabilisiert wird. Durch den hohen Materialeinsatz wird ein solches Stäbchen relativ schwer und ist in der Herstellung aufwendig. Auch birgt ein lose eingebrachter Stützstab im Falle des Bruches des Stäbchens erhöhte Verletzungsgefahr.

Das DE-Patent DE 17 41 279 U beschreibt ein Stäbchen für Wattetupfer. Der Stiel wird aus zusammengedrehter Watte hergestellt und zum Zwecke der Erhöhung der Steifigkeit mit einem leimartigen Überzug versehen. Eine weitere Erhöhung der Steifigkeit des Stieles wird durch eine Einlage aus Perlondraht oder einem anderen geeigneten Kunststoff erreicht. Diese Methode führt zwar zu einer Erhöhung der Steifigkeit, hat jedoch den Nachteil eines hohen Materialeinsatzes. Dies führt zu einer Gewichtserhöhung.

Die DE 1 183 641 A1 beschreibt hohle Kunststoffstäbchen zur Verwendung als Wattestäbchen.

Die DE 1 188 775 A1 offenbart Wattestäbchen, deren Schaft vollständig aus Kunststoff besteht, wobei jedoch eine Variation im Durchmesser oder ein zweiteilig ausgeführter Schaft vorgesehen ist.

Die FR 2 129 143 A2 offenbart Wattestäbchen mit hohlem Schaft, wobei jedoch keine Innenstege in Querrichtung vorgesehen sind. Zur Längsverstärkung sind Strukturen vorhanden, die nach der dortigen Figur 4 auch helikal ausgerichtet sein können.

Strangextrudierte Stäbchen sind hohl **(Abb. 2)** und dadurch relativ leicht.

Gängige Maße für strangextrudierte Ohrwattestäbchen, auf die sich die Erfindung bezieht, haben eine Länge von 70 bis 73 mm und einen Außendurchmesser von 1,5 bis 2,8 mm. Der Innendurchmesser verändert sich mit zu- oder abnehmendem Stäbchengewicht.

Die Hauptunterschiede zwischen Ohrwattestäbchen und Hospitalstäbchen sind lediglich die Abmessungen der Stäbchen und, daß bei Hospitalstäbchen meist nur an ein Ende des Stäbchens Watte gewickelt wird **(Abb. 3).** Gängige Durchmesser für Hospitalstäbchen sind ca. 1,5 mm bis 6,0 mm. Die Stäbchenlängen reichen von ca. 100 mm bis 400 mm. Ein Beispiel für ein gebräuchliches Hospitalstäbchen ist 140 mm Länge bei 4,0 mm Außendurchmesser.

Beide Stäbchenarten brauchen zur problemlosen Weiterverarbeitung eine gewisse Biegesteifigkeit, damit sie sich nicht zu stark verbiegen und dadurch aus der Führung der Wickelmaschine springen.

Die heute üblichen Stäbchengewichte sind nötig, um ausreichende Steifigkeit für die Weiterverarbeitung zu erzielen und um ausreichend viel Material zum "Einsiegeln" zu haben.

Einsiegeln bedeutet, daß das Stäbchen im Bereich der Enden zackenförmig angeschmolzen wird, damit anschließend die Watte an dem noch weichen Kunststoff kleben bleibt, um dann in Form gewickelt zu werden. **(Abb. 3)**

Dieses Einsiegeln kann das hohle Stäbchen durch Kerbwirkung oder "Durchsiegeln" soweit schwächen, daß es bereits beim Wickeln des Watteköpfchens oder später bei der Benutzung, eventuell im Ohr, abbrechen kann. **(Abb. 4)**

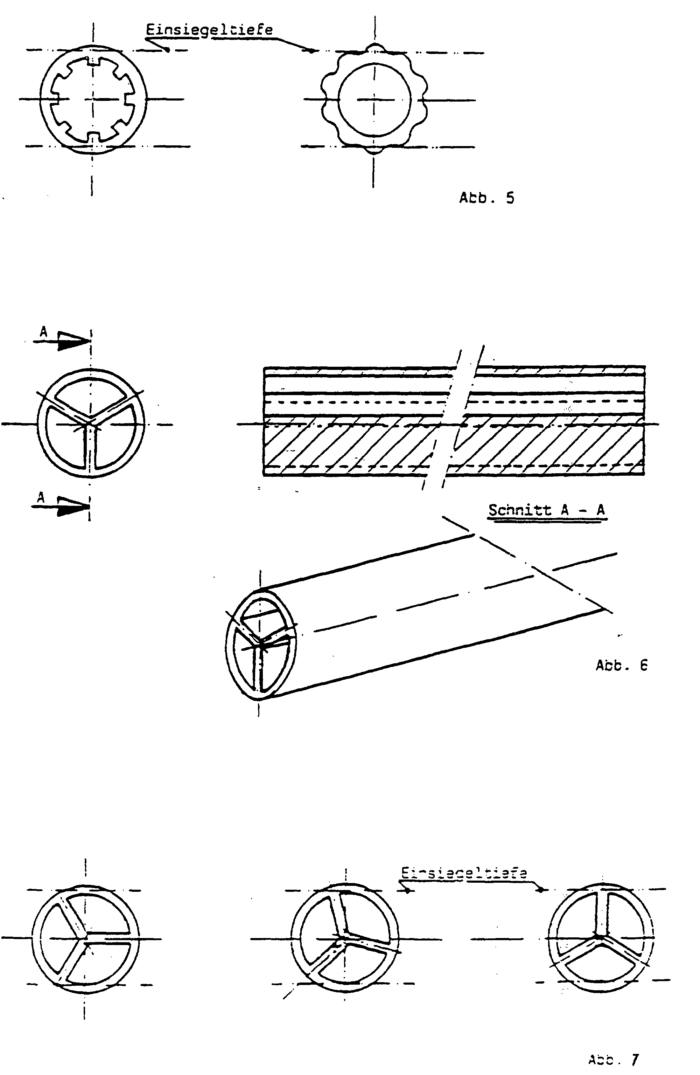

Diese Gefahren bestehen insbesondere bei den dünnwandigen Ohrwattestäbchen. Es gab in der Vergangenheit Versuche, die Stäbchengewichte durch Verringern der Wandstärke zu reduzieren und das "Durchsiegeln" mit Riffeln an der Innen- oder Außenseite des Stäbchens ( Abb. 5 ) zu verhindern. Diese Profile führten jedoch nicht zu einer Erhöhung der Biegesteifigkeit. Dies machte eine weitere Reduzierung des Stäbchengewichtes unter die heute marktgängigen Gewichte bei gleicher Verarbeitbarkeit für Ohrwattestäbchen unmöglich.

Bei der Weiterverarbeitung von Hospitalstäbchen ergeben sich ähnliche Probleme wie bei den Ohrwattestäbchen. Durch die größeren Wandstärken, die bei Hospitalstäbchen üblich sind, ist die Gefahr des-Durchsiegelns **(Abb. 4)** hier allerdings nicht so groß wie bei den Ohrwattestäbchen.

### Vorteile der Erfindung

Das Stäbchen mit Innenstegen **(Abb. 6)** löst sowohl das Problem der abnehmenden Biegesteifigkeit bei reduziertem Gewicht als auch das Problem des Abbrechens der Köpfchen durch das Einsiegeln.

Durch das geschlossene Innenprofil reißt das eingesiegelte Stäbchen mit der geringeren Wandstärke nicht mehr ab, auch wenn die Außenwand durchgesiegelt wurde, weil mindestens ein Steg immer unversehrt bleibt und die beiden anderen Stege nur angesiegelt werden können. **(Abb. 7)**

Durch den dreieckigen Verbund der Innenstege, die mit der inneren Mantelfläche durchgehend fest verbunden sind, entsteht eine gute Steifigkeit bei deutlich reduzierter Wandstärke. Die Steifigkeit nimmt zwar leicht ab, aber in Folge der Wickelbewegung beim Anbringen der Watte wird dieses Defizit dadurch ausgeglichen, daß fortlaufend die stabilisierenden Stege ein Abknicken verhindern. Gewichtseinsparungen von 15 bis 55 % im Vergleich zu den heute gängigen Gewichten werden so realisiert.

Durch Reduzierung des Gewichtes läßt sich in erster Linie Gewicht einsparen, was Ressourcen schont und dem Verbraucher Einkaufsvorteile bringt. Nebeneffekte sind aber auch geringere Karton- und Palettengewichte.

## Patentansprüche

1. Ohrwattestäbchen und Hospitalstäbchen, **dadurch gekennzeichnet, dass** das Stäbchen strangextrudiert ist mit einem durchgehenden Innensteg.

2. Ohrwattestäbchen und Hospitalstäbchen nach Anspruch 1, **dadurch gekennzeichnet, dass** der Innensteg mit der inneren Mantelfläche einfach oder mehrfach, durchgehend oder abschnittsweise durch Stege aus demselben Material verbunden ist.

## Claims

1. Cotton ear swab and hospital swab device, **characterised in that** the rod is extruded with a continuous internal web.

2. Cotton ear swab and hospital swab device as claimed in Claim 1, **characterised in that** the internal web is connected once or several times, continuously or in sections, to the inner surface of the shell by webs made from the same material.

## Revendications

1. Cotons-tiges et bâtonnets pour hôpitaux, **caractérisés en ce que** le bâtonnet est extrudé en continu avec une traverse intérieure continue.

2. Cotons-tiges et bâtonnets pour hôpitaux suivant la revendication 1, **caractérisés en ce que** la traverse intérieure est reliée à la surface d'enveloppe intérieure de manière simple ou multiple, continue ou par sections, par des traverses du même matériau.
